(19) Europäisches Patentamt
European Patent Office
Office européen des brevets

(11) **EP 2 323 692 B1**

(12) **EUROPEAN PATENT SPECIFICATION**

(45) Date of publication and mention
of the grant of the patent:
**24.04.2013 Bulletin 2013/17**

(51) Int Cl.:
*A61K 45/06* (2006.01)   *A23L 2/52* (2006.01)
*A61P 25/32* (2006.01)

(21) Application number: **09788305.2**

(86) International application number:
**PCT/NL2009/050533**

(22) Date of filing: **04.09.2009**

(87) International publication number:
**WO 2010/027264 (11.03.2010 Gazette 2010/10)**

(54) **HANGOVER RELIEF BY COMPOSITIONS COMPRISING ORAL REHYDRATION SOLUTION**

KATER-LINDERUNG DURCH ZUSAMMENSETZUNGEN, DIE EINE ORALE REHYDRATIONSLÖSUNG ENTHALTEN

COMPOSITION CONTENANT UNE SOLUTION ORALE DE REHYDRATATION, DESTINEE A DIMINUER LA VEISALGIE

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **04.09.2008 EP 08163642**

(43) Date of publication of application:
**25.05.2011 Bulletin 2011/21**

(73) Proprietor: **Early Bird Drinks B.V.**
**2311 XN Leiden (NL)**

(72) Inventors:
• **VERMEULEN, Louis**
**NL-1093 JD Amsterdam (NL)**

• **VERHOEFF, Joost, Jacobus, Cornelis**
**NL-3844 JB Harderwijk (NL)**

(74) Representative: **Swinkels, Bart Willem**
**Nederlandsch Octrooibureau**
**J. W. Frisolaan 13**
**2517 JS Den Haag (NL)**

(56) References cited:
**WO-A-02/052959      WO-A-2007/056176**
**WO-A-2008/011915**

• **DATABASE WPI Week 200781 Thomson Scientific, London, GB; AN 2007-874162 XP002520271 & CN 1 977 899 A (YAN Z) 13 June 2007 (2007-06-13)**

Printed by Jouve, 75001 PARIS (FR)

**Description**

**Background**

A hangover describes the unpleasant physiological effects following heavy alcohol consumption. The most commonly reported symptoms of a hangover include headache, nausea, and a diminished feeling of general well-being. The symptoms vary from person to person, and occasion to occasion, usually beginning several hours after drinking. It is believed that the economic impact of hangovers is severe, and estimates run as high as 2-5% of the gross-national product, mostly due to decreased productivity the day after extensive alcohol intake (Wiese et al. (2000). Ann. Intern. Med. 132, 897).

Pathophysiology of hangover is largely unknown. Hypoglycemia, dehydration, acetaldehyde intoxication and vitamin B 12 deficiency are all theorised causes of hangover symptoms. It is clear from studies that during the hangover period at least dehydration, cytokine dysregulation and gastrointestinal disturbances occur, and levels of anti-diuretic hormone and prostaglandins are elevated (Verster (2008). Alcohol Alcohol 43, 124; Pittler et al. (2005). BMJ 331, 1515). In addition to alcohol, impurities present in alcoholic beverages seem to contribute to the severity of alcohol symptoms. A wide spectrum of potential cures and preventives have been studied, with very limited, if any effect (Wiese *et al.*, *supra,* and Pittler *et al., supra).*

**Summary of the Invention**

The present inventors have now found that fast replenishment of fluids and electrolytes by ingestion of a composition comprising a modified oral rehydration solution after more than moderate alcohol consumption reduces hangover symptoms.

Thus, the invention relates to a composition comprising an oral rehydration solution.

In a further aspect, the invention relates to a composition comprising an oral rehydration solution for use as a medicament, in particular for preventing or treating one or more symptoms of a hangover.

**Description of the Drawings**

The invention will be further elucidated referring to the following figures:

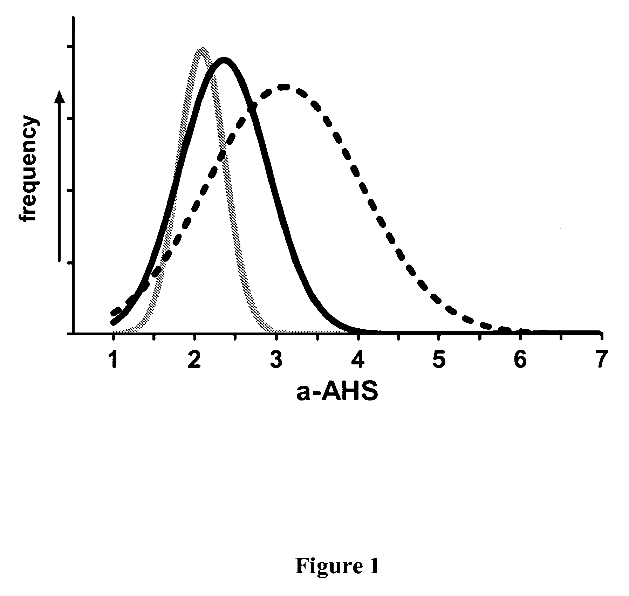

Fig. 1a shows the distribution of hangover severity as measured by a-AHS for the control (grey line), for placebo-treated group (dashed line) and for ORS-treated group (black line). Data are represented as histograms normalised for bin displaying highest number (% of maximum) in every group. The lines display Gaussian curves based on the data. Figure 1b shows that overlay of Gaussian curves illustrates apparent reduced hangover symptoms after ORS intake. Categories (none, mild, severe and very severe hangover) were deducted from comparison of a-AHS score and experienced hangover severity of placebo-treated participants.

Fig. 2 shows that ORS relieves hangover symptoms at different levels of alcohol intake. In both categories, 1.0-2.49‰ and 2.5-4.0‰ estimated blood alcohol concentrations (BAC), respectively, hangover symptoms, as indicated by a-AHS, are reduced at a similar rate.

Fig. 3 shows subjective score for items loss of appetite and nausea of the a-AHS after consumption of ORS or ORS+Yacon-syrup (ORS+Y).

**Detailed Description of Embodiments**

The present inventors have found that an oral rehydration solution may be used to prevent or treat one or more symptoms of a hangover. Thus, the present invention relates to an oral rehydration solution or a composition comprising an oral rehydration mixture, in particular for preventing or treating one or more symptoms of a hangover.

Symptoms of a hangover include, without limitation, thirst, headache, nausea, tiredness, loss of appetite, stomach ache, dizziness, faintness, and the like.

An oral rehydration solution ("ORS") is an aqueous liquid comprising a glucose containing saccharide and sodium ions. The ORS has a total osmolarity of from 200 to 350 mosmol/L. Preferably, the ORS comprises from 35 to 90 milliequivalents per Liter (meq/L) of sodium (Na[1]) and from 1.2 to 3.0 weight percent (wt %) of a glucose containing saccharide.

The glucose containing saccharide may be glucose, or a saccharide that can be hydrolyzed to form a composition containing glucose. Further examples of such glucose containing saccharides are dextrose, fructooligosaccharides, fructose, com syrup, high fructose com syrup, sucrose, maltodextrin, and mixtures thereof.

The ORS may further include potassium. The concentration of potassium ions may, for example, be in the range of 0 to 40 mmol/L. The ORS may further comprise chloride ions. The concentration of chloride ions may be in the range of 0 to 100 mmol/L. The ORS may further comprise citrate and/or bicarbonate.

The ORS may also comprise one ore more artificial sweeteners. Examples of such artificial sweeteners are sodium saccharine, aspartame, acesulfame-K and chlorinated sucrose.

The ORS may also comprise one or more additional ingredients.
Examples of additional ingredients include flavourants, colourants, preservatives, dietary supplements, and the like.

The oral rehydration solution is advantageously prepared by combining the nonaqueous ("dry") ingredients with an aqueous liquid, e.g. water, to provide a solution having the appropriate concentrations of ingredients. The dry ingredients may be combined with the aqueous liquid simultaneously, or one or more of the dry ingredients may be added separately. It may be desirable to provide all or most of the dry ORS ingredients in a single composition, referred to as an oral rehydration mixture ("ORM"). In particular, an ORM may comprise the glucose containing saccharide and sodium ions, and optionally also further ingredients used to prepare the oral rehydration solution according to the present invention.

The composition of the present invention may further comprise one or more booster additive selected from the group consisting of Yacón Syrop and vitamins.

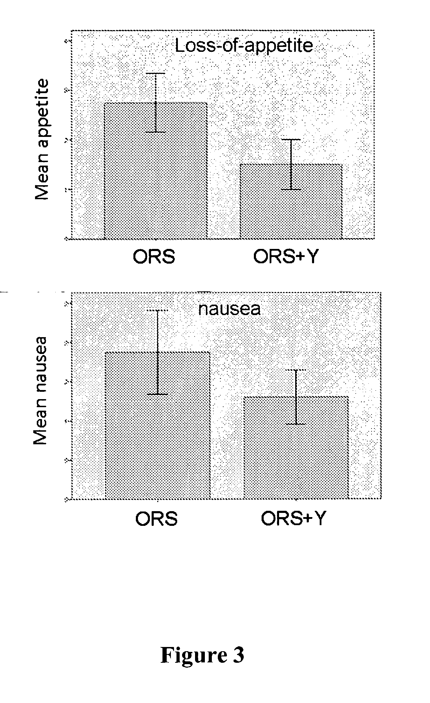

In an embodiment, the booster additive is Yacón syrup. Yacón syrup is a sweetening agent extracted from the tuberous roots of the yacón plant (Smallanthus sonchifolius), indigenous to the Andes mountains. It contains high levels of fructooligosaccherides (FOS) (Valentova,K., Sersen,F., and Ulrichova,J. (2005). Radical scavenging and anti-lipoperoxidative activities of Smallanthus sonchifolius leaf extracts. J. Agric. Food Chem. 53, 5577-5582). It is reported that FOS derived from Yacon can lower blood sugar levels and therefore, the present inventors speculated, relief nausea related symptoms (Aybar,M.J., Sanchez Riera,A.N., Grau,A., and Sanchez,S.S. (2001). Hypoglycemic effect of the water extract of Smallantus sonchifolius (yacon) leaves in normal and diabetic rats. J. Ethnopharmacol. 74, 125-132; Genta, S., Cabrera,W., Habib,N., Pons,J., Carillo,I.M., Grau,A., and Sanchez,S. (2009). Yacon syrup: beneficial effects on obesity and insulin resistance in humans. Clin. Nutr. 28, 182-187). In addition, extracts from the Yacon root contain high amounts of anti-oxidants that might be beneficial in prevention and/or recovery from the hangover syndrome and might reduce late-time effects of more then moderate alcohol consumption (Yan,X., Suzuki,M., Ohnishi-Kameyama,M., Sada, Y., Nakanishi,T., and Nagata,T. (1999). Extraction and identification of antioxidants in the roots of yacon (Smallanthus sonchifolius). J. Agric. Food Chem. 47, 4711-4713; Valentova et al., *supra*). It was observed by the present inventors that loss of appetite was significantly less in subjects receiving ORS comprising Yacon syrup compared to subject receiving ORS not comprising Yacon syrup. A similar, but small, trend was observed for the item nausea (see Figure 3). ORS comprising Yacon syrup seems to be a better and more robust anti hangover product than is ORS. Finally, we would like to emphasize that addition of Yacon-syrup to ORS enhances the palatability of the product.

The composition according to the present invention may further comprise vitamine C. Vitamine C may be added as an antioxidant to scavenge free radicals in the body, e.g. resulting from alcohol ingestion.

It has been hypothesized that alcohol may cause vitamin deficiencies, in particular of B vitamins. B vitamins are needed to help the body cope with stress, including physical stress from exposure to ethanol, its metabolites, methanol (found in red wine and cognac), and sulphites (used to preserve many beverages). Riboflavin (vitamin B2) helps the body to use oxygen and regulates the metabolism of amino acids, fatty acids, and carbohydrates. Riboflavin is further needed to activate pyridoxine (vitamin B6). Pyridoxine (vitamin B6) balances the hormonal changes in women as well as assisting the immune system and the growth of new cells. Pyridoxine also assists in the processing and metabolism of proteins, fats and carbohydrates and with controlling mood and behaviour. Cyanocobalamine (vitamin B12) guides the manufacture and maintenance of red blood cells, stimulates appetite, promotes growth and releases energy.

Thus, in an embodiment the composition according to the present invention further comprises vitamin B2, vitamin B6 and/or vitamin B12 to prevent the occurrence of vitamin deficiencies after heavy alcohol consumption.

In a further aspect, the present invention relates to a composition as described above for use as a medicament, in particular for preventing or treating one or more symptoms of a hangover. The one or more symptoms preferably include at least headache, nausea and stomach ache.

The composition according to the present invention may be further used to prevent or treat any condition involving dehydration, e.g. diarrhoea, shock, and the like.

It will be clear to the skilled person that the above description and drawings are included to illustrate the invention.

**Examples**

**Example 1**

*Materials and Methods*

Prior to participation all subjects were asked for informed consent. Solutions were prepared with mineral water (Bar-Le-Duc, Utrecht, The Netherlands).

The ORS used (Orisel®, Nutricia, Zoetermeer, The Netherlands) has the following composition: glucose 20.0 g/L, Na+: 2.06 g/L, K+: 0.79 g/L, Cl-: 2.83 g/L; osmolarity: 320 mOsmol/L. The taste of both ORS and placebo-solution was masked with aspartame (4 g/L in ORS or 6 g/L in placebo solution). Solutions were bottled in HDPE 500 mL bottles (FLEPL1072, Bloklandpack, IJsselstein, The Netherlands).

The Acute Hangover Scale as described by Rohscnow *et al.* (Rohscnow et al. (2007). Addict. Behav. 32, 1314) was adapted for the purpose. The adapted-AHS (a-AHS) consists of the average of eight items that can be scorcd from 1 (absent) up to 7 (incapacitating) : 1. hangover 2. thirsty 3. tiredness 4. headache 5. loss of appetite 6. stomach ache 7. nausea 8. dizziness, faintness. From the original AHS the inventors omitted the item 'heart racing' because it was very rare in our study population and changed the scaling in order to get a more robust indication of hangover severity. The effect sizes of Control group versus Placebo group match with first description of AHS, confirming the validity of our a-AHS. We asked the participants of the study to fill out a questionnaire shortly after waking up the morning after the trial. The control group was asked to fill out a similar score sheet on a 'regular' morning.

*Blood/Breath Alcohol Concentration:*

We obtained an indication of blood alcohol levels by assessing the volunteers' breath alcohol percentage (AlcoScan Pro, AL7000, ACE Germany).

*Statistics:*

Statistics were performed with SPSS 15.0 for windows and GraphPad Prism 4.0 as described in **Table S1.** For effect size evaluation we calculated Cohen *d* values; $d = \dfrac{mean_1 - mean_2}{\sqrt{SD_1^2 + SD_2^2 / 2}}$. Cohen roughly defined effect sizes as "small, *d*=0.2" "medium, d=0.5" and "large, d=0.8". (J. Cohen, Statistical power analysis for the behavioural sciences, 1988, 2nd ed.)

*Results*

Trial participants were recruited at social events where relatively high levels of alcohol are consumed. At arrival, when sober, potential participants were informed on trial procedures and gave informed consent. On leaving the event, subjects were randomly given either 500 ml ORS sweetened with aspartame or an equivalent amount of aspartame sweetened water. The present inventors used aspartame to minimize taste differences in order to ascertain the blind nature of the study. In addition, the present inventors established a blood alcohol concentration of a subset (n=39) of our participants at the moment they left the party (range 1.10-3.60‰). For characteristics of participants see **Table 1.** Subjects were asked to fill out an online questionnaire the morning after, to evaluate hangover severity (see **Table 1**). For this purpose the Acute Hangover Scale (AHS) as previously described by Rohsenow *et al.* (Rohsenow et al. (2007). Addict. Behav., vol. 32:1314) was adapted. The adapted-AHS (a-AHS) averages eight items (i.e. headache, thirst, nausea) that can be scored from 1 (absent) up to 7 (incapacitating).

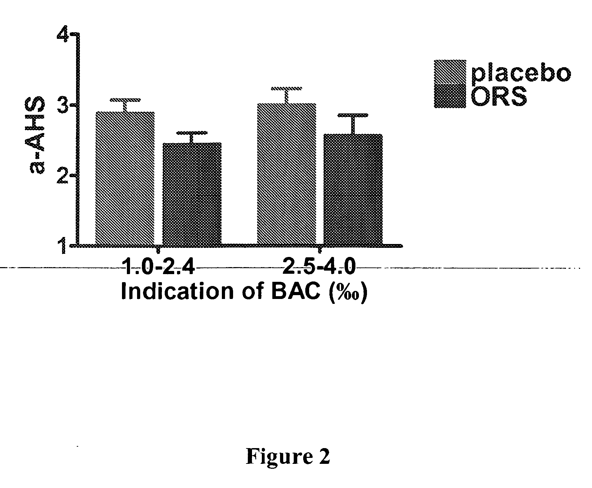

The inventors established that intake of ORS after alcohol consumption significantly reduced the hangover severity in our studied population (mean a-AHS score 3.04 in placebo-treated population and 2.39 for ORS-treated group, p<0.001) (**Fig 1**). This was mainly due to reduced scores on the items 'headache' (p=0.007) and 'stomach ache' (p=0.013). To investigate if the marked relief we found is of importance for general hangover experience the inventors asked the subjects how they would rate their hangover compared to previous ones experienced. Here the inventors also found a significant reduction (see **Table 1**; p<0.001). In addition, we calibrated the a-AHS by inclusion of a control population not subjected to extensive alcohol consumption the night before. Strikingly, intake of ORS reduces the a-AHS almost to calibrated baseline levels (**Fig 1**). An unexpected finding was a significantly higher response rate in the ORS-treated group as compared to the placebo group (78% vs. 60%; p=0.035). This difference was only observed in the male population (79% vs. 48%; p=0.004), and could indicate an increase in general performance levels. It was also found that ORS relieves hangover symptoms at different levels of alcohol intake (**Fig 2**). In both categories, 1.0-2.4‰ and 2.5-4.0‰ estimated blood alcohol concentrations (BAC), respectively, hangover symptoms, as indicated by a-AHS, were reduced at a similar level.

In conclusion, drinking 500 ml of ORS prior to sleep after extensive alcohol consumption is beneficial for prevention of hangover associated symptoms. This supports the idea that dehydration is a pivotal pathophysiological mechanism in hangover occurrence, which has until now, never been scientifically proven (Verster. (2008). Alcohol Alcohol, vol. 43:124). In addition, this might provide an easy, cheap and safe way of reducing the discomfort of the hangover, and associated public expenses. It did not escape our notice, however, that knowledge of the preventive effect of ORS might increase alcohol intake, and therefore further research to this end is warranted.

*Table 1 Participant Characteristics and Results*

| Characteristics | Control | Placebo | ORS | Significance | |
|---|---|---|---|---|---|
| *Volunteers (n)* | 52 | 67 | 59 | N.A. | |
| *Age (yr.) (mean±SD)* | **21.3** ±2.1 | **23.7** ±6.1 | **22.0** ±2.6 | p=0.401 [†] | |
| *Sex (% male)* | 52.3 | 59.7 | 64.4 | p=0.28[‡] | |
| *BAC\* (mean ‰) (range)* | **N.A.** | **2.30** (1.10-3.60) | **2.13** (1.60-3.20) | p=0.387[†] | |
| **Results[#]** | **Control** | **Placebo** | **ORS** | **Effect size (control vs. placebo)** $^{\Omega}$ | **Effect size (placebo vs. ORS)** $^{\Omega}$ |
| *Thirsty* | **3.60** ±1.47 | **3.75** ±1.19 | **3.04** ±1.40 | **0.11;** p=0.036 | **0.55;** p=0.013 |
| *Headache* | **1.63** ±1.03 | **3.30** ±1.74 | **2.35** ±1.43 | **1.17;** p<0.001 | **0.60;** p=0.007 |
| *Nausea* | **1.31** ±0.64 | **2.08** ±1.33 | **1.67** ±0.97 | **0.74;** p<0.001 | **0.35;** p=0.110 |
| *Tiredness* | **3.81** ±1.46 | **4.15** ±1.46 | **3.74** ±1.42 | **0.23;** p=0.850 | **0.28;** p=0.191 |
| *Loss of appetite* | **1.81** ±1.46 | **2.63** ±1.58 | **2.24** ±1.46 | **0.54;** p=0.149 | **0.26;** p=0.243 |
| *Hangover* | **1.00** ±N.A. | **3.55** ±1.32 | **2.59** ±1.11 | **2.73;** p<0.001 | **0.79;** p<0.001 |
| *Stomach ache* | **1.23** ±0.47 | **2.43** ±1.55 | **1.70** ±1.11 | **1.05;** p<0.001 | **0.54;** p=0.013 |
| *Dizziness, faintness* | **1.40** ±0.57 | **2.48** ±1.49 | **1.87** ±1.11 | **0.96;** p<0.001 | **0.46;** p=0.034 |
| *a-AHS score* | **1.97** ±0.43 | **3.04** ±0.90 | **2.39** ±0.59 | **1.52;** p<0.001 | **0.85;** p<0.001 |
| *Hangover severity compared to previous ones* | **N.A.** | **3.58** ±1.06 | **2.63** ±1.12 | **N.A.** | **0.87;** p<0.001 |

\* Indication of Blood Alcohol Concentration as derived from breath alcohol concentration prior to treatment in a subset

[†]Student's T-test for significance between Placebo and ORS-treated groups (p-value)

$ Fraction of volunteers that completed the on-line response form the morning after

‡ $\chi^2$ -test for significance between Placebo and ORS groups (p-value)

$^{\Omega}$Effect size calculation (Cohen d value; p-value Student's T-test) d=0.50-0.79 medium effect, d>0.8 large effect (see supplemental material and methods for details.)

[#] Items of the adapted-Acute Hangover Scale (a-AHS) (mean ±SD). Data based on volunteers that completed the online response form

**Example 2**

A formulation of ORS further comprising Yacon-syrup was tested in a population of 18 healthy young volunteers that were administered 500 ml of either ORS or ORS + 1% Yacon-syrup (ORS[+Y]) after an evening of high alcohol intake at a social event. The next morning the volunteers filled out the online a-AHS form and both groups were compared.

A significant reduction in the score on the item loss of appetite was observed in the ORS[+Y] treated group compared to the control population (Fig. 3). A similar, but small, trend was observed for the item nausea. Based on these results it is concluded that $ORS^{+Y}$ is a better and more robust anti hangover product than is ORS. Finally, addition of Yacon-syrup to conventional ORS enhanced the palatability of the product considerably.

**Claims**

1. Composition comprising an aqueous liquid comprising a glucose containing saccharide and sodium ions and Yacón Syrop.

2. Composition according to claim 1, further comprising vitamin C.

3. Composition according to claim 1 or 2, further comprising vitamin B2.

4. Composition according to any one of the preceding claims, further comprising vitamin B6.

5. Composition according to any one of the preceding claims, further comprising vitamin B12.

6. Composition according to any of claims 1-5 for use as a medicament.

7. Composition according to any of claims 1-5 for use as a medicament for preventing or treating one or more symptoms of a hangover.

8. Use of an oral rehydration solution, preferably a composition according to any of claims 1-5, in the preparation of a medicament for preventing or treating one or more symptoms of a hangover.

**Patentansprüche**

1. Zusammensetzung, die eine wässrige Flüssigkeit, die ein glukosehaltiges Saccharid und Natriumionen umfasst, sowie Yacón-Sirup umfasst.

2. Zusammensetzung nach Anspruch 1, die des Weiteren Vitamin C umfasst.

3. Zusammensetzung nach Anspruch 1 oder 2, die des Weiteren Vitamin B2 umfasst.

4. Zusammensetzung nach einem der vorangehenden Ansprüche, die des Weiteren Vitamin B6 umfasst.

5. Zusammensetzung nach einem der vorangehenden Ansprüche, die des Weiteren Vitamin B12 umfasst.

6. Zusammensetzung nach einem der Ansprüche 1-5 zur Verwendung als ein Medikament.

7. Zusammensetzung nach einem der Ansprüche 1-5 zur Verwendung als ein Medikament zum Verhindern eines Katers oder zum Behandeln eines Symptoms oder mehrerer Symptome desselben.

8. Verwendung einer oralen Rehydrations-Lösung, vorzugsweise einer Zusammensetzung nach einem der Ansprüche 1-5, bei der Herstellung eines Medikaments zum Verhindern eines Katers oder zum Behandeln eines Symptoms oder mehrerer Symptome desselben.

**Revendications**

1. Composition comprenant un liquide aqueux comprenant un saccharide contenant du glucose et des ions sodium et du sirop de Yacon.

2. Composition selon la revendication 1, comprenant de plus de la vitamine C.

3. Composition selon la revendication 1 ou 2, comprenant de plus de la vitamine B2.

4. Composition selon l'une quelconque des revendications précédentes, comprenant de plus de la vitamine B6.

5. Composition selon l'une quelconque des revendications précédentes, comprenant de plus de la vitamine B 12.

6. Composition selon l'une quelconque des revendications 1 à 5 pour son utilisation comme médicament.

7. Composition selon l'une quelconque des revendications 1 à 5 pour son utilisation comme médicament pour la prévention ou le traitement d'un ou plusieurs symptômes de la gueule de bois.

8. Utilisation d'une solution de réhydratation par voie orale, de préférence une composition selon l'une quelconque des revendications 1 à 5, dans la préparation d'un médicament pour la prévention ou le traitement d'un ou plusieurs symptômes de la gueule de bois.

**Figure 1**

**Figure 2**

**Figure 3**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **WIESE et al.** *Ann. Intern. Med.,* 2000, vol. 132, 897
- **VERSTER.** *Alcohol Alcohol,* 2008, vol. 43, 124
- **PITTLER et al.** *BMJ,* 2005, vol. 331, 1515
- **VALENTOVA,K. ; SERSEN,F. ; ULRICHOVA,J.** Radical scavenging and anti-lipoperoxidative activities of Smallanthus sonchifolius leaf extracts. *J. Agric. Food Chem.,* 2005, vol. 53, 5577-5582
- **AYBAR,M.J ; SANCHEZ RIERA,A.N. ; GRAU,A. ; SANCHEZ,S.S.** Hypoglycemic effect of the water extract of Smallantus sonchifolius (yacon) leaves in normal and diabetic rats. *J. Ethnopharmacol.,* 2001, vol. 74, 125-132
- **GENTA,S. ; CABRERA,W. ; HABIB,N. ; PONS,J. ; CARILLO,I.M. ; GRAU,A. ; SANCHEZ,S.** Yacon syrup: beneficial effects on obesity and insulin resistance in humans. *Clin. Nutr,* 2009, vol. 28, 182-187
- **YAN,X. ; SUZUKI,M. ; OHNISHI-KAMEYAMA,M. ; SADA,Y. ; NAKANISHI,T. ; NAGATA,T.** Extraction and identification of antioxidants in the roots of yacon (Smallanthus sonchifolius. *J. Agric. Food Chem.,* 1999, vol. 47, 4711-4713
- **ROHSCNOW et al.** *Addict. Behav.,* 2007, vol. 32, 1314
- **J. COHEN.** Statistical power analysis for the behavioural sciences. 1988
- **ROHSENOW et al.** *Addict. Behav.,* 2007, vol. 32, 1314